# EUROPÄISCHE PATENTSCHRIFT

(11) **EP 3 077 359 B1**
(45) Veröffentlichungstag und Bekanntmachung des Hinweises auf die Patenterteilung: **27.02.2019**
(21) Anmeldenummer: 14814765.5
(22) Anmeldetag: 05.12.2014
(51) Int. Cl.: C07C 51/02, C07C 51/09, C07C 51/43, C07C 51/493, C07C 55/10, C07C 67/08, C07C 69/40, C12P 7/62

(54) **VERFAHREN ZUR HERSTELLUNG UND ISOLIERUNG VON CARBONSÄUREESTERN**
PROCESS FOR THE PREPARATION AND ISOLATION OF CARBOXYLIC ESTERS
PROCÉDÉ DE PRODUCTION ET D'ISOLEMENT D'ESTERS D'ACIDE CARBOXYLIQUE

(30) Priorität: 06.12.2013 DE 102013225215
(43) Veröffentlichungstag der Anmeldung: 12.10.2016
(73) Patentinhaber: thyssenkrupp Industrial Solutions AG, 45143 Essen (DE); thyssenkrupp AG, 45143 Essen (DE)
(72) Erfinder: FRITSCH, Markus, 04229 Leipzig (DE); BÖRNER, Armin, 18059 Rostock (DE); SHUKLOV, Ivan, 18059 Rostock (DE); KNEZ, Zeljko, SI-2000 Maribor (SI)
(74) Vertreter: thyssenkrupp Intellectual Property GmbH
(86) Internationale Anmeldenummer: PCT/EP2014/003254
(87) Internationale Veröffentlichungsnummer: WO 2015/082077

(56) Entgegenhaltungen:
- US-A1- 2005 070 738
- US-A1- 2009 234 160
- US-A1- 2011 245 515
- US-A1- 2011 275 851
- DATABASE WPI Week 200540 Thomson Scientific, London, GB; AN 2005-389773 XP002735589, & JP 2005 132836 A (NATIONAL INSTITUTE OF ADVANCED INDUSTRIAL SCIENCE AND TECHNOLOGY) 26. Mai 2005 (2005-05-26)
- DATABASE CAPLUS [Online] CHEMICAL ABSTRACTS SERVICE, COLUMBUS, OHIO, US; XP002735590, Database accession no. 2012:1531268 & CN 102 731 302 A (BEIJING XUYANG CHEMICAL TECHNOLOGY INSTITUTE) 17. Oktober 2012 (2012-10-17)

## Beschreibung

Die Erfindung betrifft ein Verfahren zur Herstellung und Isolierung von Carbonsäureestern. Dieses Verfahren basiert auf der Umsetzung einer Carbonsäure mit einem Alkohol im wässrigen Medium. Hierfür wird der Alkohol sowohl zur Veresterung als auch zur Ausfällung der bei der Synthese entstehenden Salze, bevorzugt Ammoniumsalze, eingesetzt.

Aus dem Stand der Technik sind verschiedene Verfahren zur Herstellung von Carbonsäuren durch fermentative Prozesse bekannt, z. B. für Bernsteinsäure, Milchsäure oder Zitronensäure. Für optimale Prozessbedingungen im Fermenter erfolgt eine Einstellung des pH-Wertes der Fermentationsbrühe durch Zusatz einer Base (z.B. Ammoniumhydroxid, Ammoniumbicarbonat, Natriumhydroxid, Calciumhydroxid, etc.). In Abhängigkeit vom pH-Wert kommt es dann zur Bildung eines Carbonsäuresalzes, z. B. Diammoniumsuccinat bei der Neutralisation von Bernsteinsäure mit einer AmmoniumBase, oder einer Mischung aus Carbonsäure und Salz.

Es ist aus dem Stand der Technik bekannt, Carbonsäuresalze z. B. durch Elektrodialyse wieder in die freie Carbonsäure zu überführen. Allerdings sind derartige Verfahren mit einem hohen Energieverbrauch verbunden und neigen dazu, dass es zu einem Faulen auf der Oberfläche der Membranen kommt, wodurch die Lebensdauer der Membranen stark eingeschränkt wird.

Andere Prozesse bedienen sich eines Ansäuerungsschrittes, in dem durch Zugabe einer starken Säure die Carbonsäure isoliert wird, während das sich dabei bildende Carbonsäuresalz in Lösung (im Falle von Ammoniumsulfat) bzw. in der Suspension (im Falle von Calciumsulfat, sog. "Gips-Verfahren") verbleibt. Bei der Ansäuerung von Diammoniumsuccinat mit Schwefelsäure kommt es so zur Bildung von Ammoniumsulfat, einem wertvollen Düngemittel.

Um eine Trennung der Carbonsäure von dem Salz zu erreichen, erfolgt beim "Gips-Verfahren" eine Filtration. Hierbei ist aber von Nachteil, dass der gebildete Gips ein Abfallprodukt darstellt und nicht weiterverwertet werden kann.

Eine weitere Variante für die Trennung von Carbonsäure und Salz basiert auf einer chromatographischen Trennung, z. B. einer kontinuierlichen Chromatographie (SMB, "Simulated Moving Bed"). Durch die hohen apparativen Kosten für die chromatographische Einheit und den hohen Verbrauch an Wasser als Elutionsmittel für eine effiziente Trennung von Salz und Säure treten hier aber verfahrensökonomische Nachteile auf. Ebenso ist es nachteilig, dass aufgrund der langen Verweilzeiten bei hoher Temperatur, die für die Verdampfung des Wassers erforderlich sind, in der Regel eine Verfärbung des Salzes eintritt, die auf Reaktionen der verbliebenen Aminosäuren und der Zucker im salzhaltigen Raffinatstrom bei der Chromatographie zurückzuführen sind.

Für die Rückgewinnung der Carbonsäure oder Carbonsäureanhydride in der gewünschten Reinheit sind weitere Schritte erforderlich. Hierzu zählen gängige Technologien wie lonenaustausch, Nanofiltration, Umkehrosmose, Extraktion, Verdampfung, Destillation, Kristallisation oder Rekristallisation. Je höher hier die Anforderungen an die Reinheit der Carbonsäure sind, desto größer sind allerdings auch der mit der Reinigung verbundene Aufwand und die Einbußen hinsichtlich der Ausbeute.

Im Falle der Bernsteinsäure ist hier die wichtigste Anwendung in der Herstellung von 1,4-Butandiol (BDO), Tetrahydrofuran (THF) und γ-Butyrolacton (GBL) zu sehen. Letzteres stellt das Ausgangsmaterial für die Herstellung von 2-Pyrrolidon dar.

BDO, THF und GBL können durch einen Veresterungs- und Hydrierungsprozess ausgehend von Maleinsäureanhydrid hergestellt werden, dem DAVY-Prozess. Ein Zwischenprodukt in diesem Verfahren stellt das Dimethylsuccinat (DMSAC) dar. Da dieses durch eine Veresterung von Bernsteinsäure hergestellt wird, könnte DMSAC in ein konventionelles Hydrierungsverfahren für die Herstellung von BDO, THF oder GBL eingespeist werden.

Um wettbewerbsfähig mit vergleichbaren konventionellen Ausgangsmaterialien zu sein, ist es erforderlich, den Prozess der Isolierung und Reinigung der fermentierten Ausgangsmaterialien möglichst effizient zu gestalten. Im Falle von Bernsteinsäure und dessen Derivaten gelten die Reinigung und Kristallisation der Bernsteinsäure und des Bernsteinsäureanhydrids und die anschließenden Schritte der Auflösung in Methanol, Veresterung und Hydrierung gelten aufgrund der hohen Anzahl an Prozessschritten, des Energieverbrauchs und der vielen Phasenübergänge als Schwachpunkte hinsichtlich der Effizienz.

Weiterhin ist es bekannt, dass die Verdampfungskristallisation von Carbonsäuren, wie Bernsteinsäure, einen sensiblen Prozess darstellt, der die erreichbare Reinheit der Kristalle und die Menge an Verunreinigungen durch Einschlüsse oder Sorptionseffekte beeinflusst. Es kann daher erforderlich sein, eine Kristallisation/Rekristallisation anzuschließen, um die Verunreinigungen auf ein für die folgende Veresterung verträgliches Maß zu senken.

Üblicherweise werden bei fermentativen Prozessen neben der gewünschten Carbonsäure auch weitere Carbonsäuren als Nebenprodukte gebildet, die nur mit großem Aufwand durch die zuvor genannten Trennverfahren entfernt werden können. Für die fermentative Gewinnung von Bernsteinsäure kommt es beispielsweise gleichzeitig zur Bildung von u.a. Essigsäure, Milchsäure, Fumarsäure und Maleinsäure als Nebenprodukten. In Abhängigkeit von den Spezifikationen für die Bernsteinsäure für den Veresterungs- und Hydrierungsschritt zur Bildung von DMSAC oder sogar für die nachfolgende Herstellung von Bio-Polymeren wie Polybutylensuccinat (PBS) kann die Anreicherung dieser weiteren Carbonsäuren als Nebenprodukte zur Bildung von unerwünschten Alkoholen oder Estern führen.

Eine große Hürde für biotechnologische Prozesse stellt die Menge des eingesetzten Wassers dar. Dies betrifft die energieeffiziente Abtrennung des Produkts, die große Menge an erzeugtem Abwasser und das Erfordernis katalytischer Reaktionen in wässriger Umgebung.

Für die Bereitstellung effizienter Prozesse muss in Abhängigkeit vom Endprodukt das Lösungsverhalten der Zielkomponenten berücksichtigt und katalytische Prozesse angepasst werden.

Die US 2011/0275851 A1 offenbart ein Verfahren zur Herstellung von Diethylsuccinat, wobei Natrium- oder Calciumsuccinat aus einer Fermentationsbrühe zuerst angesäuert, das gebildete Natrium- oder Calciumsulfat entfernt und die resultierende freie Bernsteinsäure verestert wird.

Die US 2009/0234160 A1 offenbart ein Verfahren zur Herstellung von freier Bernsteinsäure durch Ansäuerung eines Alkalimetallsuccinats.

Die US 2005/0070738 A1 offenbart ein Verfahren zur Herstellung einer freien organischen Säure A, (z.B. Bernsteinsäure), bei der man eine Säure B (z.B. Essigsäure) zum Ammoniumsalz der organischen Säure zugibt, um das Ammoniumsalz der Säure B und die freie Säure A zu bilden.

Die US 2011/0245515 A1 offenbart ein Verfahren zur Überführung von Diammoniumsuccinat aus einer Fermentationsbrühe in freie Bernsteinsäure durch Destillation, Kristallisation und wieder Destillation.

In Database WPI, AN 2005-389773 & JP 2005-132836 (National Institute of Advanced Industrial Science and Technology), 26. Mai 2005, wird ein Verfahren zur Desaminierung von Ammoniumsuccinat unter Bildung von Bernsteinsäure durch Reaktion mit Alkohol oder Wasser beschrieben, wobei das Ammoniak zurückgewonnen wird.

Database CAplus, AN 2012:1531268 & CN 102731302 A (Beijing Xuyang Chemical Technology Institute), 17. Oktober 2012, offenbart ein Verfahren zur Herstellung von Dimethylsuccinat durch Veresterung von Diammoniumsuccinat.

Ausgehend von diesen aus dem Stand der Technik bekannten Nachteilen war es Aufgabe der vorliegenden Erfindung, ein Verfahren zur Herstellung und Isolierung von Carbonsäureestern bereitzustellen, dass zum einen eine hohe Produktreinheit gewährleistet und zum anderen den technischen Aufwand für die einzelnen Verfahrensschritte minimiert.

Diese Aufgabe wird durch das Verfahren mit den Merkmalen des Anspruchs 1 gelöst. Die weiteren abhängigen Ansprüche zeigen vorteilhafte Weiterbildungen auf.

Erfindungsgemäß wird ein Verfahren zur Herstellung und Isolierung von Carbonsäureestern von Mono- und Dicarbonsäuren, Hydroxycarbonsäuren und Fettsäuren bereitgestellt, das folgende Schritte aufweist.
a) Bereitstellung einer wässrigen Lösung mindestens eines Carbonsäuresalzes,
b) Ansäuerung der wässrigen Lösung mit mindestens einer Säure unter Bildung der freien Carbonsäure und eines Salzes der Säure,
c) Fällung des Salzes durch Zugabe mindestens eines Alkohols zur Lösung,
d) Abtrennung des ausgefällten Salzes aus der Lösung,
e) Veresterung der mindestens einen freien Carbonsäure durch Zugabe mindestens eines Alkohols und
f) Abtrennung des mindestens einen Carbonsäureesters von der Lösung,
wobei bei der Veresterung in Schritt e) ein Katalysator zugegeben wird, der ausgewählt ist aus
- der Gruppe wasserunlöslicher Säuren, insbesondere Dodecylbenzolsulfonsäure,
- der Gruppe von Lipasen,
- der Gruppe fester Säuren oder
- Mischungen hiervon.

Sofern im Folgenden von einer Carbonsäure gesprochen wird, ist hierunter immer mindestens eine Carbonsäure zu verstehen. Es kann sich somit auch um eine Mischung mehrerer Carbonsäuren handeln.

Besonderes Merkmal des erfindungsgemäßen Verfahrens ist es dabei, dass auf einfache Weise eine Abtrennung der Salze der bei der Ansäuerung zugesetzten Säure von der freien Carbonsäure in wässriger Lösung erfolgt, indem durch Zugabe eines Alkohols eine Ausfällung des Salzes erfolgt, das im Anschluss dann mit technisch einfachen Mitteln von der wässrigen Lösung der freien Carbonsäure abgetrennt werden kann.

Mit dem erfindungsgemäßen Verfahren sind dabei die folgenden wesentlichen Vorteile gegenüber den aus dem Stand der Technik bekannten Verfahren verbunden:
- Herstellung von Carbonsäureestern hoher Reinheit
- Herstellung von Salzen, z.B. Ammoniumsalzen, als Nebenprodukten in hoher Reinheit
- Verringerung der Gefahr des biologischen Foulings
- Energieeffiziente Verfahrensweise durch die Reduzierung der Wasserströme im Prozess, einschließlich des Abwassers
- Das Verfahren erlaubt ein Rezyklierung der Lösungsmittel

Vorzugsweise handelt es sich bei dem Salz um ein Ammoniumsalz, das durch Zugabe von Ammoniumhydroxid oder Ammoniumbicarbonat als Base erzeugt werden kann.

Es ist aber auch möglich andere Salze der Carbonsäure bereitzustellen, indem als Base z.B. Natrium-, Kalium-, Calciumhydroxid oder Mischungen hiervon eingesetzt werden.

Das erfindungsgemäße Verfahren eignet sich bevorzugt für Prozesse, bei denen die Carbonsäure fermentativ gebildet wird. In diesem Fall liegt die Carbonsäure in einer Fermentationsbrühe vor. Durch Neutralisation mit einer Base kommt es zur Bildung des im Schritt a) eingesetzten Carbonsäuresalzes.

Wurde die Carbonsäure fermentativ hergestellt, so ist es bevorzugt, dass vor, während oder nach dem Schritt b) in einem weiteren Verfahrensschritt eine Abtrennung der Biomasse, z.B. von Zellen, Zellbestandteilen und Proteine, erfolgt. In diesem Verfahrensschritt können beispielsweise auch weitere Feststoffe abgetrennt werden, sofern diese in der Fermentationsbrühe anwesend sind. Hinsichtlich der Trennverfahren sind alle gängigen aus dem Stand der Technik bekannten Trennungsmethoden möglich. Hierzu zählen beispielsweise die gravimetrische Trennung, die Zentrifugation, die Mikro-, Ultra- oder Nanofiltration sowie Kombinationen von den genannten Trennmethoden.

Ebenso ist es möglich, dass die Carbonsäure durch andere Biotransformationsverfahren bereitgestellt wurde.

Eine weitere bevorzugte Variante des erfindungsgemäßen Verfahrens sieht vor, dass vor Schritt b), d.h. vor der Neutralisierung der wässrigen Lösung, eine Aufkonzentration der Lösung erfolgt. Dies kann bevorzugt durch Umkehrosmose oder durch Eindampfen der Lösung realisiert werden.

Hinsichtlich der zur Ansäuerung eingesetzten Säure in Schritt b) sind alle Protonensäuren bevorzugt, die einen pKₛ-Wert aufweisen, der kleiner als der pKₛ -Wert der zu isolierenden Carbonsäure ist. Diese Protonensäure ist vorzugsweise ausgewählt aus der Gruppe bestehend aus Schwefelsäure, Phosphorsäure, Salpetersäure, Salzwasser, Königswasser, Kohlensäure sowie Mischungen hiervon.

Es ist weiter bevorzugt, dass der in Schritt c) für die Fällung des Salzes der Säure zugegebene Alkohol ausgewählt ist aus der Gruppe
- der geradkettigen oder verzweigten C₁-C₈-Alkohole, insbesondere Methanol, Ethanol, Propanol, Isopropanol, Butanol, Isobutanol, Pentanol, Hexanol, Heptanol, Octanol und Mischungen hiervon,
- der Gruppe der geradkettigen oder verzweigten C₁-C₈-Diolen, insbesondere Ethylenglykol, Propandiol, Butandiol, Pentandiol, Hexandiol, Heptandiol, Octandiol und Mischungen hiervon,
- der Gruppe der geradkettigen oder verzweigten C₁-C₈-Polyolen sowie
- Mischungen hiervon.

Die Abtrennung des ausgefällten Salzes in Schritt d) kann durch beliebige Trennmethoden erreicht werden. Bevorzugt sind hier die gravimetrische Trennung, die Zentrifugation oder Kombinationen hiervon.

Das abgetrennte Salz kann im Anschluss vorzugsweise gewaschen und/oder getrocknet werden, sodass auch diese Salze für eine Weiterverarbeitung genutzt werden können.

Hinsichtlich des Veresterungsschrittes e) wird vorzugsweise ein Verhältnis von Alkohol zu Wasser von 1:5 bis 10:1, bevorzugt 1:2 bis 5:1, besonders bevorzugt 1:1 bis 5:1 eingestellt.

Bei der Veresterung wird ein Katalysator zugegeben, der ausgewählt ist aus der
- der Gruppe wasserunlöslicher Säuren, insbesondere Dodecylbenzolsulfonsäure,
- der Gruppe von Lipasen wie Novozym 435 oder Amano PS
- der Gruppe fester Säuren wie Amberlyst 15 oder
- Mischungen hiervon.

Für den Fall, dass eine wasserlösliche Säure als Katalysator eingesetzt wird, ist es besonders bevorzugt, dass es sich hierbei um die identische Säure handelt, die in Schritt b) bei der Ansäuerung zugegeben wird. Hierdurch wird eine Rückführung der Säure im Prozess ermöglicht, was besonders verfahrensökonomisch ist. In diesem Fall erfolgt die Veresterung in Schritt e) vorzugsweise bei einer Temperatur von 5°C bis 90°C, bevorzugt 10°C bis 60°C und besonders bevorzugt von 20°C bis 50°C und/oder einem Druck von 0,1 bis 10 bar, bevorzugt 0,5 bis 5bar und besonders bevorzugt 1 bis 2 bar.

Eine weitere bevorzugte Variante sieht vor, dass der in Schritt e) zugegebene Alkohol ausgewählt ist aus
- der Gruppe der geradkettigen oder verzweigten C₁-C₈-Alkohole, insbesondere Methanol, Ethanol, Propanol, Isopropanol, Butanol, Isobutanol, Pentanol, Hexanol, Heptanol, Octanol und Mischungen hiervon,
- der Gruppe der geradkettigen oder verzweigten C₁-C₈-Diolen, insbesondere Ethylenglykol, Propandiol, Butandiol, Pentandiol, Hexandiol, Heptandiol, Octandiol und Mischungen hiervon,
- der Gruppe der geradkettigen oder verzweigten C₁-C₈-Polyolen sowie
- Mischungen hiervon.

Auch hinsichtlich des eingesetzten Alkohols ist es besonders bevorzugt, dass der in Schritt e) für die Veresterung eingesetzte Alkohol identisch mit dem für die Ausfällung des Salzes der Säure im Schritt c) zugegebene Alkohol ist. Auch dies resultiert in einer besonders verfahrensökonomischen Technik, da der Alkohol rezykliert wird.

Es ist weiter bevorzugt, dass bei der Veresterung in Schritt e) die Carbonsäureester extrahiert werden. Dies kann bevorzugt mit organischen Lösungsmitteln, insbesondere Toluol, Chloroform, MTBE oder über- bzw. unterkritischen Fluiden erfolgen. Besonders bevorzugt ist hier der Einsatz von überkritischen CO₂. Auch hier kann eine verfahrensökonomische Rückführung der Extraktionsmittel in den Prozess erfolgen.

Besonders bevorzugt ist eine Variante, bei der die Schritte der Fällung und Abtrennung des gefällten Salzes der Säure sowie der Veresterung, d.h. die Schritt c), d) und e) simultan erfolgen.

Die in Schritt f) vorgesehene Abtrennung der Carbonsäureester von der Lösung kann vorzugsweise mittels Destillation oder durch chromatographische Verfahren erfolgen. Unter den chromatographischen Verfahren sind dabei insbesondere unter- oder überkritische flüssigchromatographische Verfahren bevorzugt. In diesem Trennungsschritt kann, wenn verschiedene Carbonsäureester vorliegen, auch eine Trennung dieser Carbonsäureester voneinander erfolgen.

Grundsätzlich ist das erfindungsgemäße Verfahren für alle Mono- und Dicarbonsäuren, Hydroxycarbonsäuren und Fettsäuren geeignet. Unter den Mono- oder Dicarbonsäuren sind beispielsweise Ameisensäure, Essigsäure, Propionsäure, Buttersäure, Valeriansäure, Capronsäure, Oxalsäure, Malonsäure, Bernsteinsäure, Glutarsäure, Adipinsäure, Maleinsäure, Fumarsäure, Sebazinsäure, Dodekandisäure, Itaconsäure und Mischungen hiervon zu nennen. Die Hydroxycarbonsäuren sind vorzugsweise ausgewählt aus der Gruppe bestehend aus Apfelsäure, Glykolsäure, Mandelsäure, Milchsäure, Tartronsäure, Weinsäure, Zitronensäure, 3-Hydroxypropionsäure, Hydroxybuttersäure, Mevalonsäure, Gallussäure, Salicylsäure, Hydroxybenzoesäure und Mischungen hiervon.

Eine Ausführungsform der Erfindung sieht vor, dass die Veresterung in Schritt e) bei einer Temperatur von 5°C bis 150°C, bevorzugt 30°C bis 100°C und besonders bevorzugt von 50°C bis 90°C und/oder einem Druck von 0,1 bis 10 bar, bevorzugt 0,5 bis 5 bar und besonders bevorzugt 1 bis 2 bar erfolgt.

Eine Ausführungsform der Erfindung sieht vor, dass bei der Veresterung in Schritt e) CO₂ als Katalysator zugegeben wird und die Veresterung in Schritt e) bei einer Temperatur von 5°C bis 90°C, bevorzugt 10°C bis 60°C und besonders bevorzugt von 20°C bis 50°C und/oder einem Druck von 1 bis 300 bar, bevorzugt 20 bis 200 bar und besonders bevorzugt 80 bis 120 bar erfolgt.

Anhand der nachfolgenden Beispiele und Figuren soll der erfindungsgemäße Gegenstand näher erläutert werde, ohne diesen die hier gezeigten spezifischen Ausführungsformen einschränken zu wollen.

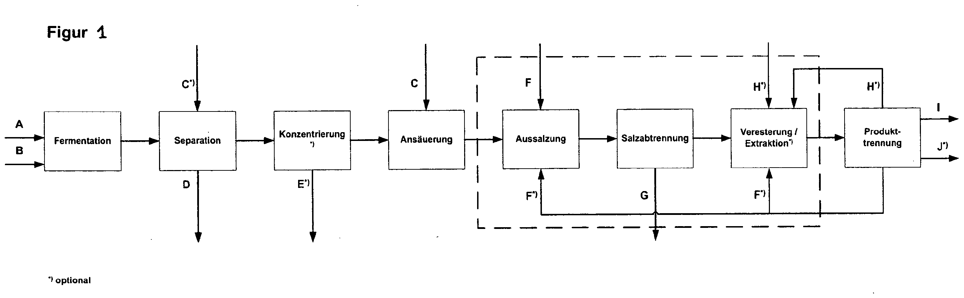

Die Figur zeigt anhand einer schematischen Darstellung eine Ausführungsform des erfindungsgemäßen Verfahrens.

In der Figur ist eine Ausführungsform des erfindungsgemäßen Verfahrens dargestellt, bei der zunächst eine fermentative Herstellung der Carbonsäure erfolgt. Hierbei wird zunächst eine Fermentationsbrühe A aus Wasser, Medienbestandteilen und Zucker vorgelegt, in der die Bildung der Carbonsäure erfolgt. Durch Zugabe von Ammoniumhydroxid B kommt es dann zur Bildung des Ammoniumsalzes. Im folgenden Separationsschritt wird dann die Biomasse D, z. B. Zellen, Zellbestandteile und Proteine, abgetrennt. In diesem Schritt kann zusätzlich Schwefelsäure zugegeben werden.

Der sich anschließende optionale Konzentrierungsschritt kann durch Eindampfen der wässrigen Lösung des Ammoniumsalzes der Carbonsäure erfolgen, wodurch eine Wasserabtrennung bewirkt wird.

Im folgenden Schritt erfolgt dann eine Ansäuerung der wässrigen Lösung durch Zugabe von Schwefelsäure. Hierbei kommt es zur Bildung der freien Carbonsäure und des Ammoniumsalzes der Säure. Im Aussalzungsschritt wird dann durch Zugabe eines Alkohols F die Ausfällung des Ammoniumsulfats bewirkt. Nachfolgend wird dann das ausgefällte Ammoniumsulfat G von der Lösung z.B. durch Zentrifugation oder gravimetrische Trennung abgetrennt. Anschließend folgt der Veresterungsschritt, bei dem durch Zugabe eines Alkohols F die freie Carbonsäure verestert wird. Dieser Schritt kann mit einer Extraktion kombiniert werden, indem ein Extraktionsmittel H zugesetzt wird.

Abschließend folgt dann der Schritt der Produkttrennung, bei der eine Abtrennung des Carbonsäureesters I von der Lösung erfolgt. Weiterhin werden das Abwasser J, der Alkohol F und gegebenenfalls das Extraktionsmittel H voneinander getrennt. Der Alkohol F und das Extraktionsmittel H können dem Prozess bei den betroffenen Verfahrensschritten wieder zugeführt werden, was besonders verfahrensökonomisch ist.

Die Verfahrensschritte der Aussalzung, der Salzabtrennung und der Veresterung können in separaten Einheiten erfolgen. Ebenso ist es aber auch möglich diese Schritte in Teileinheiten miteinander beliebig zu kombinieren bzw. auch in einer einzigen Einheit durchzuführen.

### Beispiel 1

### Veresterung von wässrigen Lösungen von Bernsteinsäure

Es erfolgte eine Umsetzung von Bernsteinsäure mit Methanol im wässrigen Medium. Die Reaktion erfolgte in einem zweiphasigen Medium und wurde mit Dodecylbenzolsulfonsäure (DBSA) bzw. Lipasen (Novozym 435, Armano PS) katalysiert. In Gegenwart von DBSA bildete sich mit Bernsteinsäure eine homogene Lösung.

Die Veresterung in technischem Methanol verlief schon bei 60°C schnell und fast quantitativ (Tabelle 1, run 1) die Reaktion in einem 1:1-Gemisch von Methanol/H₂O als Lösungsmittel verlief langsamer. Das Gleichgewicht wurde nach 24 Stunden erreicht. Es liegt bei ca. 50 % Dimethylsuccinat im Gemisch. Die Katalyse mit Novozym 435 in alkoholischer oder wässriger Lösung war weniger effizient (Runs 5 bis 10). Die maximale Ausbeute von 45 % wurde in reinem Methanol nach 48 Stunden erreicht.

**Tabelle 1**

| Run | Katalysator | Losungsmittel | Zeit [h] | Ausbeute^{d} [%] |
|---|---|---|---|---|
| 1 | DBSA^{b} | MeOH | 6 | 99 |
| 2 | DBSA^{b} | MeOH:H₂O 1:1 | 6 | 40 |
| 3 | DBSA^{b} | MeOH:H₂O 1:1 | 24 | 50 |
| 4 | DBSA^{b} | MeOH:H₂O 1:1 | 48 | 50 |
| 5 | Novozym 435^{c} | MeOH | 6 | 5 |
| 6 | Novozym 435^{c} | MeOH | 24 | 23 |
| 7 | Novozym 435^{c} | MeOH | 48 | 45 |
| 8 | Novozym 435^{c} | MeOH:H₂O 1:1 | 6 | 2 |
| 9 | Novozym 435^{c} | MeOH:H₂O 1:1 | 24 | 10 |
| 10 | Novozym 435^{c} | MeOH:H₂O 1:1 | 48 | 20 |

| | | | | |
|---|---|---|---|---|
| ^{a}3 g Bernsteinsäure, 30 mL Losungsmittel, 60 °C; ^{b}1g DBSA; ^{c}120 mg Novozym 435; ^{d}GC-Aasbeute. | | | | |

### Beispiel 2

### Veresterung von Bernsteinsäure in Anwesenheit eines organischen Lösungsmittels

Die Reaktion von Bernsteinsäure mit Methanol wurde in Gegenwart von DBSA in einem zweiphasigen 3-Komponenten-System (Methanol, H₂O/organisches Lösungsmittel) untersucht. Als organische Lösungsmittel wurden hier Chloroform (CHCl₃), Methyl-tert-Butylether (MTBE) und Toluol, die unter den Reaktionsbedingungen inert sind, eingesetzt.

Das Reaktionsgemisch mit MTBE bildet unter den Reaktionsbedingungen kein Zweiphasengemisch. Die GC-Analyse zeigte, dass die Veresterung in diesem System sehr langsam abläuft (Tabelle 2, run 5).

**Tabelle 2**

| Run | Katalysator | Lösungsmittel | Zeit [h] | Ausbeute^{d} [%] | |
|---|---|---|---|---|---|
| | | | | org. Phase | wässrige Phase |
| 1 | DBSA^{b} | CHCl₃ | 7 | 55 | 5 |
| 2 | DBSA^{b} | Toluol | 7 | 50 | 15 |
| 3 | DBSA^{b} | CHCl₃ | 20 | 78 | 4 |
| 4 | DBSA^{b} | Toluol | 20 | 56 | 22 |
| 5 | DBSA^{b} | MTBE | 7 | 1 | - |
| 6 | Amberlyst 15 | CHCl₃ | 7 | 3 | n.d. |
| 7 | Amberlyst 15 | Toluol | 7 | 25 | n.d. |
| 8 | Amberlyst 15 | CHCl₃ | 14 | 32 | 1 |
| 9 | 15 | Toluol | 14 | 47 | 15 |
| 10 | H₂SO₄ | Toluol | 7 | 59 | n.d. |
| 11 | H₂SO₄ | Toluol | 20 | 64 | 16 |

| | | | | | |
|---|---|---|---|---|---|
| ^{a}2 g Bernsteinsäure, 20 mL Lösungsmittel, 10 mL H₂O,65 °C; ^{b}0.7 g DBSA; ^{c}120 mg Novozym 435; ^{d} GC-Ausbeute an DMSAC. | | | | | |

Die beiden anderen Lösungsmittel CHCl₃ und Toluol bilden eine wässrige Phase (Methanol/H₂O) und eine organische Phase (Methanol/organisches Lösungsmittel). Schon nach sieben Stunden wurden in beiden Gemischen mehr als 50 % Ausbeute festgestellt (Tabelle 2, Runs 1 bis 2). Das Veresterungsprodukt verteilte sich zwischen den beiden Phasen.

Die GC-Ausbeuten an Produkt, die aus den Konzentrationen von Dimethylsuccinat (DMSAC) in organischer und wässriger Phasen berechnet wurden, sind in Tabelle 2 zusammengefasst. Die Verteilung des Produktes zwischen organischem und wässrigem Medium war in Chloroform besser als in Toluol. Auch die freie Säure und Monomethylsuccinat (MMSAC) wurden effektiv in die organische Phase extrahiert.

Toluol war deutlich selektiver und löst praktisch keine Bernsteinsäure und MMSAC. Die beste Ausbeute von 78 % wurde bei Anwendung von CHCl₃ und DBSA als Katalysator nach 20 Stunden bei 65°C erreicht (Run 3).

Die Verwendung von anderen Brønsted-Säuren, nämlich Amberlyst 15 und Schwefelsäure, wurde ebenfalls untersucht. Beide Säuren konnten die Veresterung katalysieren. Im Fall des stark sauren Kationenharzes Amberlyst 15 bildet sich ein 3-PhasenSystem bestehend aus Harz/org. Phase/wässrige Phase, daher sind die Ausbeuten sehr stark von der Rühreffizienz abhängig (Runs 6-9).

Die Verwendung von Schwefelsäure ist ebenfalls möglich (Runs 10-11). Hier verblieb die Säure praktisch ausschließlich in der wässrigen Phase.

### Beispiel 3

### Ausfällung und Isolierung von Ammoniumsulfat (erfindungsgemäße Schritte c und d)

5ml einer 10g/L konzentrierten Diammoniumsuccinat-Lösung wurden mit Schwefelsäure auf pH 2,2 angesäuert.

Die hierbei erzeugte wässrige Lösung aus Ammoniumsulfat und Bernsteinsäure wurde im volumetrischen Verhältnis von einem Teil dieser wässrigen Lösung zu 4 Teilen Methanol bei Raumtemperatur gemischt. Der hierbei ausgefallene Rückstand wurde mit einer Massenausbeute von 70% (bezogen auf die theoretisch erzeugte Menge Ammoniumsulfat) isoliert und mittels ¹H-, ¹³C-NMR-Spektroskopie und Elementaranalyse untersucht.

Die Analyse des Rückstandes ergab praktisch reines Ammoniumsulfat mit ca. 0,5 % Verunreinigungen an Bernsteinsäure.

Das Ergebnis zweier Elementaranalysen war hierbei:
C=0.3816%,H=6.046%, N=21.35%,S=23.30%
C=0.2327%,H=6.271%,N=21.48%,S=24.21%

Die Analyse abgedampften Mutterlauge ergab ein Gemisch aus Bernsteinsäure und geringen Resten an Ammoniumsulfat (ca. 15 %).

Das Ergebnis zweier Elementaranalysen war hierbei:
C=31.07%,H=4.931 %,N=3.471%,S=2.521%
C=31.73%,H=5.038%,N=3.171%,S=3.684%

## Patentansprüche

1. Verfahren zur Herstellung und Isolierung von Carbonsäureestern von Mono- und Dicarbonsäuren, Hydroxycarbonsäuren und Fettsäuren mit folgenden Schritten: a) Bereitstellung einer wässrigen Lösung mindestens eines Salzes mindestens einer Carbonsäure,
b) Ansäuerung der wässrigen Lösung mit mindestens einer Säure unter Bildung der freien Carbonsäure und eines Salzes der Säure, c) Fällung des Salzes durch Zugabe mindestens eines Alkohols zur Lösung,
d) Abtrennung des ausgefällten Salzes aus der Lösung,
e) Veresterung der mindestens einen freien Carbonsäure durch Zugabe mindestens eines Alkohols und
f) Abtrennung des mindestens einen Carbonsäureesters von der Lösung,
wobei bei der Veresterung in Schritt e) ein Katalysator zugegeben wird, der ausgewählt ist aus
• der Gruppe wasserunlöslicher Säuren, insbesondere Dodecylbenzolsulfonsäure,
• der Gruppe von Lipasen,
• der Gruppe fester Säuren oder
• Mischungen hiervon.

2. Verfahren nach Anspruch 1,
**dadurch gekennzeichnet, dass** das Salz ausgewählt ist aus der Gruppe bestehend aus Ammoniumsalz, Calciumsalz, Kaliumsalz, Natriumsalz oder Mischungen hiervon.

3. Verfahren nach einem der vorhergehenden Ansprüche,
**dadurch gekennzeichnet, dass** in einem vorgelagerten Schritt die Carbonsäure mittels Fermentation oder anderen
Biotransformationsprozessen hergestellt wird und die wässrige Lösung in Schritt a) eine Fermentationsbrühe ist, die gegebenenfalls Biomasse enthält und somit als Suspension vorliegt, wobei durch Neutralisation mit einer Base das Carbonsäuresalz für Schritt a) hergestellt wird.

4. Verfahren nach dem vorhergehenden Anspruch,
**dadurch gekennzeichnet, dass** in einem weiteren Schritt vor, während oder nach Schritt b) eine Abtrennung der Biomasse, insbesondere Zellen, und gegebenenfalls in der Suspension enthaltenen Feststoffen aus der Suspension erfolgt, insbesondere durch ein Trennverfahren ausgewählt aus der Gruppe gravimetrische Trennung, Zentrifugation, Mikrofiltration, Ultrafiltration, Nanofiltration und Kombinationen hiervon.

5. Verfahren nach einem der vorhergehenden Ansprüche,
**dadurch gekennzeichnet, dass** vor Schritt b) eine Aufkonzentration der Lösung erfolgt, insbesondere durch Umkehrosmose oder Eindampfen der Lösung.

6. Verfahren nach einem der vorhergehenden Ansprüche,
**dadurch gekennzeichnet, dass** die in Schritt b) zugegebene Säure einen pKs-Wert aufweist, der kleiner als der pKs-Wert der zu isolierenden Carbonsäure ist, und insbesondere ausgewählt ist aus der Gruppe bestehend aus Schwefelsäure, Phosphorsäure, Salpetersäure,
Salzwasser, Königswasser, Kohlensäure sowie Mischungen hiervon.

7. Verfahren nach einem der vorhergehenden Ansprüche,
**dadurch gekennzeichnet, dass** der in Schritt c) zugegebene Alkohol ausgewählt ist aus
• der Gruppe der geradkettigen oder verzweigten C1-C8-Alkohole, insbesondere Methanol, Ethanol, Propanol, Isopropanol, Butanol, Isobutanol, Pentanol, Hexanol, Heptanol, Octanol und Mischungen hiervon,
• der Gruppe der geradkettigen oder verzweigten C1-C8-Diolen, insbesondere Ethylenglykol, Propandiol, Butandiol, Pentandiol, Hexandiol, Heptandiol, Octandiol und Mischungen hiervon,
• der Gruppe der geradkettigen oder verzweigten C1-C8-Polyolen sowie
• Mischungen hiervon.

8. Verfahren nach einem der vorhergehenden Ansprüche,
**dadurch gekennzeichnet, dass** die Abtrennung in Schritt d) durch ein Trennverfahren ausgewählt aus der Gruppe gravimetrische Trennung, Zentrifugation und Kombinationen hiervon erfolgt.

9. Verfahren nach einem der vorhergehenden Ansprüche,
**dadurch gekennzeichnet, dass** das in Schritt d) abgetrennte Salz der Säure in einem weiteren Schritt gewaschen und/oder getrocknet wird.

10. Verfahren nach einem der vorhergehenden Ansprüche,
**dadurch gekennzeichnet, dass** bei der Veresterung in Schritt e) ein Verhältnis von Alkohol zu Wasser von 1:5 bis 10:1, bevorzugt 1:2 bis 5:1 und besonders bevorzugt 1:1 bis 5:1 eingestellt wird.

11. Verfahren nach Anspruch 1, **dadurch gekennzeichnet, dass** die Veresterung in Schritt e) bei einer Temperatur von 5°C bis 150°C, bevorzugt 30°C bis 100°C und besonders bevorzugt von 50°C bis 90°C und/oder einem Druck von 0,1 bis 10 bar, bevorzugt 0,5 bis 5 bar und besonders bevorzugt 1 bis 2 bar erfolgt.

12. Verfahren nach einem der vorhergehenden Ansprüche,
**dadurch gekennzeichnet, dass** bei der Veresterung in Schritt e) CO2 als Katalysator zugegeben wird.

13. Verfahren nach dem vorhergehenden Anspruch,
**dadurch gekennzeichnet, dass** die Veresterung in Schritt e) bei einer Temperatur von 5°C bis 90°C, bevorzugt 10°C bis 60°C und besonders bevorzugt von 20°C bis 50ºC und/oder einem Druck von 1 bis 300 bar, bevorzugt 20 bis 200 bar und besonders bevorzugt 80 bis 120 bar erfolgt.

14. Verfahren nach dem vorhergehenden Anspruch,
**dadurch gekennzeichnet, dass** die wasserlösliche Säure identisch zu der in Schritt b) zugegebenen Säure ist, so dass eine Rückführung der Säure im Verfahren möglich ist.

15. Verfahren nach einem der vorhergehenden Ansprüche,
**dadurch gekennzeichnet, dass** der in Schritt e) zugegebene Alkohol ausgewählt ist aus
• der Gruppe der geradkettigen oder verzweigten C1-C8-Alkohole, insbesondere Methanol, Ethanol, Propanol, Isopropanol, Butanol, Isobutanol, Pentanol, Hexanol, Heptanol, Octanol und Mischungen hiervon,
• der Gruppe der geradkettigen oder verzweigten C1-C8-Diolen, insbesondere Ethylenglykol, Propandiol, Butandiol, Pentandiol, Hexandiol, Heptandiol, Octandiol und Mischungen hiervon, • der Gruppe der geradkettigen oder verzweigten C1-C8-Polyolen sowie
• Mischungen hiervon.

16. Verfahren nach dem vorhergehenden Anspruch,
**dadurch gekennzeichnet, dass** der in Schritt e) zugegebene Alkohol identisch zu dem in Schritt c) zugegebenen Alkohol ist, so dass eine Rückführung des Alkohols im Verfahren möglich ist,
insbesondere ein Rückführung in den Schritt c) und/oder den Schritt e).

17. Verfahren nach einem der vorhergehenden Ansprüche,
**dadurch gekennzeichnet, dass** bei der Veresterung in Schritt e) die Carbonsäureester extrahiert wenden, insbesondere mit mindestens einem organischen Lösungsmittel, insbesondere Toluol, Chloroform, MTBE oder über- oder unterkritischen Fluiden, insbesondere überkritischem CO2, das vorzugsweise in das Verfahren rückführbar ist.

18. Verfahren nach einem der vorhergehenden Ansprüche,
**dadurch gekennzeichnet, dass** die Schritte c), d) und e) simultan erfolgen.

19. Verfahren nach einem der vorhergehenden Ansprüche,
**dadurch gekennzeichnet, dass** die Trennung der Carbonsäureester untereinander und von der Lösung in Schritt f) mittels Destillation oder chromatographisch, insbesondere mittels unter- oder überkritischer Flüssigchromatographie erfolgt.

20. Verfahren nach einem der vorhergehenden Ansprüche,
**dadurch gekennzeichnet, dass** nach Schritt f) der Carbonsäureester wieder in die freie Carbonsäure überführt wird.

21. Verfahren nach einem der vorhergehenden Ansprüche,
**dadurch gekennzeichnet, dass** die Mono- oder Dicarbonsäuren ausgewählt sind aus der Gruppe bestehend aus Ameisensäure, Essigsäure, Propionsäure, Buttersäure, Valeriansäure, Capronsäure, Oxalsäure, Malonsäure, Bernsteinsäure, Glutarsäure, Adipinsäure, Maleinsäure, Fumarsäure, Sebazinsäure, Dodekandisäure, Itaconsäure und Mischungen hiervon und/oder die Hydroxycarbonsäuren ausgewählt sind aus der Gruppe bestehend aus Äpfelsäure,
Glykolsäure, Mandelsäure, Milchsäure, Tartronsäure, Weinsäure, Zitronensäure, , 3-Hydroxypropionsäure, Hydroxybuttersäure, Mevalonsäure, Gallussäure, Salicylsäure, Hydroxybenzoesäure und Mischungen hiervon.

## Claims

1. Method for preparing and isolating carboxylic esters of mono- and dicarboxylic acids, hydroxycarboxylic acids and fatty acids with the following steps:
a) providing an aqueous solution of at least one salt of at least one carboxylic acid,
b) acidifying the aqueous solution with at least one acid to form the free carboxylic acid and a salt of the acid,
c) precipitating the salt by addition of at least one alcohol to the solution,
d) removing the precipitated salt from the solution,
e) esterifying the at least one free carboxylic acid by addition of at least one alcohol and
f) separating the at least one carboxylic ester from the solution.
wherein a catalyst is added in the esterification in step e) which is selected from
• the group of water-insoluble acids, in particular dodecylbenzenesulfonic acid,
• the group of lipases,
• the group of solid acids or
• mixtures thereof.

2. Method according to Claim 1,
**characterized in that** the salt is selected from the group consisting of ammonium salt, calcium salt, potassium salt, sodium salt or mixtures thereof.

3. Method according to either of the preceding claims,
**characterized in that** the carboxylic acid is prepared in an upstream step by means of fermentation or other biotransformation processes and the aqueous solution in step a) is a fermentation broth which optionally comprises biomass and is therefore present as a suspension, wherein the carboxylic acid salt for step a) is prepared by neutralization with a base.

4. Method according to the preceding claim,
**characterized in that** in a further step, before, during or after step b), the biomass, particularly cells and solids possibly present in the suspension, is removed from the suspension, in particular by a separation process selected from the group of gravimetric separation, centrifugation, microfiltration, ultrafiltration, nanofiltration and combinations thereof.

5. Method according to any of the preceding claims, **characterized in that** the solution is concentrated before step b), particularly by reverse osmosis or evaporation of the solution.

6. Method according to any of the preceding claims,
**characterized in that** the acid added in step b) has a pKa which is less than the pKa of the carboxylic acid to be isolated and in particular is selected from the group consisting of sulfuric acid, phosphoric acid, nitric acid, salt water, aqua regia, carbonic acid or mixtures thereof.

7. Method according to any of the preceding claims,
**characterized in that** the alcohol added in step c) is selected from
• the group of straight-chain or branched C₁-C₈-alcohols, particularly methanol, ethanol, propanol, isopropanol, butanol, isobutanol, pentanol, hexanol, heptanol, octanol and mixtures thereof,
• the group of straight-chain or branched C₁-C₈-diols, particularly ethylene glycol, propanediol, butanediol, pentanediol, hexanediol, heptanediol, octanediol and mixtures thereof,
• the group of straight-chain or branched C₁-C₈-polyols and also
• mixtures thereof.

8. Method according to any of the preceding claims,
**characterized in that** the removal in step d) is effected by a separation process selected from the group of gravimetric separation, centrifugation and combinations thereof.

9. Method according to any of the preceding claims,
**characterized in that** the salt of the acid removed in step d) is washed and/or dried in a further step.

10. Method according to any of the preceding claims,
**characterized in that** the ratio of alcohol to water in the esterification in step e) is adjusted to from 1:5 to 10:1, preferably from 1:2 to 5:1 and particularly preferably from 1:1 to 5:1.

11. Method according to Claim 1,
**characterized in that** the esterification in step e) is carried out at a temperature of 5°C to 150°C, preferably 30°C to 100°C and particularly preferably 50°C to 90°C and/or a pressure of 0.1 to 10 bar, preferably 0.5 to 5 bar and particularly preferably 1 to 2 bar.

12. Method according to any of the preceding claims,
**characterized in that** CO₂ is added as catalyst in the esterification in step e).

13. Method according to the preceding claim,
**characterized in that** the esterification in step e) is carried out at a temperature of 5°C to 90°C, preferably 10°C to 60°C and particularly preferably 20°C to 50°C and/or a pressure of 1 to 300 bar, preferably 20 to 200 bar and particularly preferably 80 to 120 bar.

14. Method according to the preceding claim,
**characterized in that** the water-soluble acid is identical to the acid added in step b) such that a recycling of the acid in the method is possible.

15. Method according to any of the preceding claims, **characterized in that** the alcohol added in step e) is selected from
• the group of straight-chain or branched C₁-C₈-alcohols, particularly methanol, ethanol, propanol, isopropanol, butanol, isobutanol, pentanol, hexanol, heptanol, octanol and mixtures thereof,
• the group of straight-chain or branched C₁-C₈-diols, particularly ethylene glycol, propanediol, butanediol, pentanediol, hexanediol, heptanediol, octanediol and mixtures thereof,
• the group of straight-chain or branched C₁-C₈-polyols and also
• mixtures thereof.

16. Method according to the preceding claim,
**characterized in that** the alcohol added in step e) is identical to the alcohol added in step c) such that recycling of the alcohol in the method is possible, in particular recycling into step c) and/or step e).

17. Method according to any of the preceding claims,
**characterized in that**, in the esterification in step e), the carboxylic esters are extracted, in particular with at least one organic solvent, particularly toluene, chloroform, MTBE or supercritical or subcritical fluids, particularly supercritical CO₂, which is preferably recyclable into the method.

18. Method according to any of the preceding claims,
**characterized in that** steps c), d) and e) are carried out simultaneously.

19. Method according to any of the preceding claims,
**characterized in that** the separation of the carboxylic esters from one another and from the solution in step f) is effected by distillation or chromatographically, particularly by subcritical or supercritical fluid chromatography.

20. Method according to any of the preceding claims,
**characterized in that** after step f) the carboxylic ester is again converted into the free carboxylic acid.

21. Method according to any of the preceding claims,
**characterized in that** the mono- or dicarboxylic acids are selected from the group consisting of formic acid, acetic acid, propionic acid, butyric acid, valeric acid, caproic acid, oxalic acid, malonic acid, succinic acid, glutaric acid, adipic acid, maleic acid, fumaric acid, sebacic acid, dodecanedioic acid, itaconic acid and mixtures thereof and/or the hydroxycarboxylic acids are selected from the group consisting of malic acid, glycolic acid, mandelic acid, lactic acid, tartronic acid, tartaric acid, citric acid, 3-hydroxypropionic acid, hydroxybutyric acid, mevalonic acid, gallic acid, salicylic acid, hydroxybenzoic acid and mixtures thereof.

## Revendications

1. Procédé pour la préparation et l'isolement d'esters d'acides monocarboxyliques et dicarboxyliques, d'acides hydroxycarboxyliques et d'acides gras présentant les étapes suivantes :
a) mise à disposition d'une solution aqueuse d'au moins un sel d'au moins un acide carboxylique,
b) acidification de la solution aqueuse par au moins un acide avec formation de l'acide carboxylique libre et d'un sel de l'acide,
c) précipitation du sel par addition d'au moins un alcool à la solution,
d) séparation du sel précipité de la solution,
e) estérification dudit au moins un acide carboxylique libre par addition d'au moins un alcool et
f) séparation dudit au moins un ester d'acide carboxylique de la solution,
un catalyseur étant ajouté à l'estérification dans l'étape e), qui est choisi parmi
- le groupe des acides insolubles dans l'eau, en particulier l'acide dodécylbenzènesulfonique,
- le groupe des lipases,
- le groupe des acides solides ou
- des mélanges de ceux-ci.

2. Procédé selon la revendication 1, **caractérisé en ce que** le sel est choisi dans le groupe constitué par un sel d'ammonium, un sel de calcium, un sel de potassium, un sel de sodium ou des mélanges de ceux-ci.

3. Procédé selon l'une quelconque des revendications précédentes, **caractérisé en ce que**, dans une étape préalable, l'acide carboxylique est préparé par fermentation ou d'autres procédés de biotransformation et la solution aqueuse dans l'étape a) est un bouillon de fermentation qui contient le cas échéant une biomasse et qui se trouve donc sous forme d'une suspension, le sel d'acide carboxylique pour l'étape a) étant préparé par neutralisation avec une base.

4. Procédé selon la revendication précédente, **caractérisé en ce que**, dans une autre étape avant, pendant ou après l'étape b), une séparation de la biomasse, en particulier de cellules, et le cas échéant de solides contenus dans la suspension à partir de la suspension est effectuée, en particulier par un procédé de séparation choisi dans le groupe constitué par la séparation par gravimétrie, la centrifugation, la microfiltration, l'ultrafiltration, la nanofiltration et des combinaisons de celles-ci.

5. Procédé selon l'une quelconque des revendications précédentes, **caractérisé en ce qu'**une concentration de la solution est effectuée avant l'étape b), en particulier par osmose inverse ou par concentration par évaporation de la solution.

6. Procédé selon l'une quelconque des revendications précédentes, **caractérisé en ce que** l'acide ajouté dans l'étape b) présente une valeur pKa qui est inférieure à la valeur pKa de l'acide carboxylique à isoler et est en particulier choisi dans le groupe constitué par l'acide sulfurique, l'acide phosphorique, l'acide nitrique, l'eau salée, l'eau régale, l'acide carbonique ainsi que des mélanges de ceux-ci.

7. Procédé selon l'une quelconque des revendications précédentes, **caractérisé en ce que** l'alcool ajouté dans l'étape c) est choisi parmi
- le groupe des alcools linéaires ou ramifiés en C1-C8, en particulier le méthanol, l'éthanol, le propanol, l'isopropanol, le butanol, l'isobutanol, le pentanol, l'hexanol, l'heptanol, l'octanol et des mélanges de ceux-ci,
- le groupe des diols linéaires ou ramifiés en C1-C8, en particulier l'éthylèneglycol, le propanediol, le butanediol, le pentanediol, l'hexanediol, l'heptanediol, l'octanediol et des mélanges de ceux-ci,
- le groupe des polyols linéaires ou ramifiés en C1-C8 ainsi que
- des mélanges de ceux-ci.

8. Procédé selon l'une quelconque des revendications précédentes, **caractérisé en ce que** la séparation dans l'étape d) a lieu par un procédé de séparation choisi dans le groupe constitué par la séparation par gravimétrie, la centrifugation et des combinaisons de celles-ci.

9. Procédé selon l'une quelconque des revendications précédentes, **caractérisé en ce que** le sel de l'acide séparé dans l'étape d) est lavé et/ou séché dans une autre étape.

10. Procédé selon l'une quelconque des revendications précédentes, **caractérisé en ce qu'**on règle, lors de l'estérification dans l'étape e), un rapport alcool à eau de 1:5 à 10:1, de préférence de 1:2 à 5:1 et de manière particulièrement préférée de 1:1 à 5:1.

11. Procédé selon la revendication 1, **caractérisé en ce que** l'estérification dans l'étape e) a lieu à une température de 5°C à 150°C, de préférence de 30°C à 100°C et de manière particulièrement préférée de 50°C à 90°C et/ou à une pression de 0,1 à 10 bars, de préférence de 0,5 à 5 bars et de manière particulièrement préférée de 1 à 2 bars.

12. Procédé selon l'une quelconque des revendications précédentes, **caractérisé en ce que** du CO₂ est ajouté comme catalyseur lors de l'estérification dans l'étape e).

13. Procédé selon la revendication précédente, **caractérisé en ce que** l'estérification dans l'étape e) a lieu à une température de 5°C à 90°C, de préférence de 10°C à 60°C et de manière particulièrement préférée de 20°C à 50°C et/ou à une pression de 1 à 300 bars, de préférence de 20 à 200 bars et de manière particulièrement préférée de 80 à 120 bars.

14. Procédé selon la revendication précédente, **caractérisé en ce que** l'acide soluble dans l'eau est identique à l'acide ajouté dans l'étape b), de telle sorte qu'un recyclage de l'acide dans le procédé est possible.

15. Procédé selon l'une quelconque des revendications précédentes, **caractérisé en ce que** l'alcool ajouté dans l'étape e) est choisi parmi
- le groupe des alcools linéaires ou ramifiés en C1-C8, en particulier le méthanol, l'éthanol, le propanol, l'isopropanol, le butanol, l'isobutanol, le pentanol, l'hexanol, l'heptanol, l'octanol et des mélanges de ceux-ci,
- le groupe des diols linéaires ou ramifiés en C1-C8, en particulier l'éthylèneglycol, le propanediol, le butanediol, le pentanediol, l'hexanediol, l'heptanediol, l'octanediol et des mélanges de ceux-ci,
- le groupe des polyols linéaires ou ramifiés en C1-C8 ainsi que
- des mélanges de ceux-ci.

16. Procédé selon la revendication précédente, **caractérisé en ce que** l'alcool ajouté dans l'étape e) est identique à l'alcool ajouté dans l'étape c), de telle sorte qu'un recyclage de l'alcool dans le procédé est possible, en particulier un recyclage dans l'étape c) et/ou l'étape e).

17. Procédé selon l'une quelconque des revendications précédentes, **caractérisé en ce que** les esters d'acide carboxylique sont extraits lors de l'estérification dans l'étape e), en particulier à l'aide d'au moins un solvant organique, en particulier le toluène, le chloroforme, le MTBE ou des fluides supercritiques ou sous-critiques, en particulier le CO₂ supercritique, qui peut de préférence être recyclé dans le procédé.

18. Procédé selon l'une quelconque des revendications précédentes, **caractérisé en ce que** les étapes c), d) et e) ont lieu simultanément.

19. Procédé selon l'une quelconque des revendications précédentes, **caractérisé en ce que** la séparation des esters d'acide carboxylique les uns des autres et de la solution dans l'étape f) a lieu par distillation ou par chromatographie, en particulier par chromatographie en phase liquide sous-critique ou supercritique.

20. Procédé selon l'une quelconque des revendications précédentes, **caractérisé en ce qu'**après l'étape f), l'ester d'acide carboxylique est de nouveau transformé en acide carboxylique libre.

21. Procédé selon l'une quelconque des revendications précédentes, **caractérisé en ce que** les acides monocarboxyliques ou dicarboxyliques sont choisis dans le groupe constitué par l'acide formique, l'acide acétique, l'acide propionique, l'acide butyrique, l'acide valérianique, l'acide caproïque, l'acide oxalique, l'acide malonique, l'acide succinique, l'acide glutarique, l'acide adipique, l'acide maléique, l'acide fumarique, l'acide sébacique, l'acide dodécanedioïque, l'acide itaconique et des mélanges de ceux-ci et/ou les acides hydroxycarboxyliques sont choisis dans le groupe constitué par l'acide malique, l'acide glycolique, l'acide mandélique, l'acide lactique, l'acide tartronique, l'acide tartrique, l'acide citrique, l'acide 3-hydroxypropionique, l'acide hydroxybutyrique, l'acide mévalonique, l'acide gallique, l'acide salicylique, l'acide hydroxybenzoïque et des mélanges de ceux-ci.
